# EUROPEAN PATENT APPLICATION

(11) **EP 3 301 175 A1**
(43) Date of publication of application: **04.04.2018**
(21) Application number: 16191944.4
(22) Date of filing: 30.09.2016
(51) Int. Cl.: C12N 5/0789, C12N 15/113

(54) **MEANS AND METHODS FOR MOBILIZING HEMATOPOIETIC STEM CELLS (HSCS)**

(71) Applicant: RIJKSUNIVERSITEIT GRONINGEN, 9712 CP Groningen (NL); ACADEMISCH ZIEKENHUIS GRONINGEN, 9713 GZ Groningen (NL)
(72) Inventor: de Haan, Gerald, 9713 AV Groningen (NL)
(74) Representative: V.O.

(57) **Abstract**

The invention relates to the field of medicine, more specifically to means and methods for mobilizing hematopoietic stem cells (HSCs). Provided is a composition comprising a microRNA-125 (miR-125) nucleic acid molecule, or the complement sequence thereof, and/or an agent capable of inhibiting a target of miR-125, said target comprising in its RNA sequence the seed sequence 5'-CCUGAG-3', for use in a method for enhancing the mobilization of HSCs from the bone marrow to the peripheral blood in a subject. Also provided is a composition comprising (i) a miR-125 nucleic acid molecule, or the complement sequence thereof, and/or an agent capable of inhibiting a target of miR-125, said target comprising in its RNA sequence the seed sequence 5'-CCUGAG-3', and (ii) a further stem cell mobilization agent.

## Description

The invention relates to the field of medicine. More specifically, it relates to means and methods for mobilizing hematopoietic stem cells.

Hematopoietic stem cells (HSCs) are used in an ever-increasing number of clinical transplantation procedures. Whereas HSCs are normally only localized in the bone marrow, they can be 'mobilized' to the peripheral blood, from which they can easily harvested. Most clinical HSCs transplantations are carried out using these peripheral blood-derived HSCs. HSCs can be mobilized to the blood by multiple growth factors. Currently, stem cell mobilization in patients is typically carried out by administrating Granulocyte-Colony Stimulating Factor (G-CSF) to patients. Generally, in such treatment, patients are primed with a low dose of a chemotherapeutic agent like cyclophosphamide. During the remission, the patient is treated with a CSF, such as G-CSF, which causes eventual mobilization of cells from the bone marrow to the peripheral circulation for harvesting of leukophoresed blood. The patient is thereafter administered a high dose of chemotherapy to induce clinical remission of their cancer. The resultant bone marrow failure is treated by infusion of the stored blood cells collected previously. This procedure may be modified, e.g., by the omission of the initial dose of chemotherapy and/or alternate blood collection protocols.

While the use of these hematopoietic stem cell transplantation techniques looks promising, multiple apheresis procedures are required to harvest sufficient stem cells for successful engraftment to treat severe myelosuppression when G-CSF is used alone. Thus, despite these significant advances and the availability of certain regulatory biomolecules, delayed recovery of hematopoiesis remains an important source of morbidity and mortality for myelosuppressed patients.

There exists a continuing need in the art for compositions and methods to enhance hematopoietic recovery, particularly in cases of chemotherapy associated myelosuppression. Since there is a clear correlation between the number of stem cells that is transplanted and time to recovery for transplanted patients, it is highly relevant to mobilize, and subsequently harvest, as many stem cells as possible. Furthermore, growth factor induced stem cell mobilization is not effective in all patients, leaves many stem cells unmobilized and requires careful timing.

Therefore, improved methods to increase stem cell mobilization are of significant clinical relevance.

Micro RNAs are evolutionarily conserved, single stranded molecules of about 22 nucleotides in length and function post-transcriptionally by partial binding (partial complementarity) to the mRNA of genes. Binding of a specific miRNA to its target on an mRNA can inhibit its expression by a variety of mechanisms. The mature miRNA binds to the target mRNA and typically in the 3'-untranslated region (3'- UTR). Thus, it interferes with translation of the mRNA. MiRNAs base-pair with miRNA recognition elements (MREs) located on their mRNA targets, usually on the 3'-UTR, through a critical region called the 'seed region' which includes nucleotides 2-8 from the 5-end of the miRNA. Although the most common mechanism is translational repression as a result of miRNA binding to the 3'UTR of an mRNA, mechanisms involving mRNA degradation and destabilization have also been described. Micro RNAs are currently considered as "master regulators" of gene expression. Since a single miRNA can bind and consequently regulate the expression of more than 100 different transcripts it has been estimated that miRNAs may be able to regulate up to 30% of the protein-coding genes in the human genome.

The present inventors surprisingly found that overexpression of selected microRNA's (miR-125a, miR-125b1 or miR-125b2) in HSCs results in spontaneous stem cell mobilization, and renders stem cells highly sensitive to G-CSF. Furthermore, a large scale proteomic screens revealed protein targets that are repressed by these microRNA's, several of which can be repressed pharmacologically. Repressing these miR targets, by administrating microRNA mimetics or specific small molecule inhibitors to stem cell donors, is advantageously used to strongly enhance stem cell mobilization.

Combining the (pharmacological) inhibition of miRNA125 targets with routine stem cell mobilization regimes such as G-CSF administration is predicted to greatly increase the number of transplantable hematopoietic stem cells that can be harvested from the peripheral blood of stem cell donors.

Accordingly, in one embodiment the invention provides a composition comprising a microRNA-125 (miR-125) nucleic acid molecule, or the complement sequence thereof, and/or an agent capable of inhibiting a (direct) target of miR-125, said target comprising in its RNA sequence the seed sequence 5'-CCUGAG-3', for use in a method for enhancing the mobilization of hematopoietic stem cells (HSCs) from the bone marrow to the peripheral blood in a subject.

A composition for use as provided herein is not known or suggested in the art.

EP2460877 relates to modulating the differentiation of hematopoietic stem and progenitor cells, more particularly to the maintenance of self-renewal capability of CD133+ hematopoietic stem cells through microRNAs. EP2460877 suggests to expose human HSCs to miR sequences, including miR-125a-5p and miR-125b, prior to infusing them into patients, with the intention to achieve that the exposed cells reach the bone marrow more efficiently than non-exposed cells. The present invention aims to achieve just the opposite, namely that treated cells are released from the bone marrow, so that they start circulating in the peripheral blood. Moreover, they become very sensitive to G-CSF.

WO2005/017160 relates to methods and pharmaceutical compositions for mobilizing hematopoietic stem and progenitor cell from bone marrow into peripheral blood by administration of at least one inhibitor of a GTPase, such as Rac1 and/or Rac2 GTPase. P38 MAPK is one of the downstream targets of RacGTPAse. Inhibition of the GTPase would result in upregulation of p38MAPK activity, which is distinct from the present invention encompassing the use of a p38 inhibitory agent.

Wojtowicz *et al.* show that ectopic expression of a single miRNA, miR-125a, in purified murine and human multipotent progenitors (MPP) resulted in increased self-renewal and robust long-term multi-lineage repopulation in transplanted recipient mice. Using quantitative proteomics and Western blot analysis, a restricted set of miR-125a targets was identified which revealed the involvement of the MAP kinase signaling pathway in conferring long-term repopulating capacity to multipotent progenitors in human and mice. Importantly however, Wojtowicz *et al.* fail to teach or suggest to use the miR-125a targets in a method for enhancing the mobilization of hematopoietic stem cells from the bone marrow to the peripheral blood in a subject.

As used herein, the term "microRNA-125 nucleic acid molecule" means a single- or double-stranded nucleic acid molecule constituting microRNA-125. Throughout the specification, 'microRNA-125' is interchangeably used with 'miR-125'.

MiR-125 is a family of various microRNAs including miR-125a, 1miR-25b1 and mi-R125b2, sharing the same seed sequence. Useful in the present invention are the miR-125 homologs derived from human and non-human animals including monkeys, pigs, horses, cows, sheep, dogs, cats, mice, rabbits, and the like. Preferred examples include human microRNA-125a, microRNA-125b1 and microRNA-125b2, but are not limited thereto.

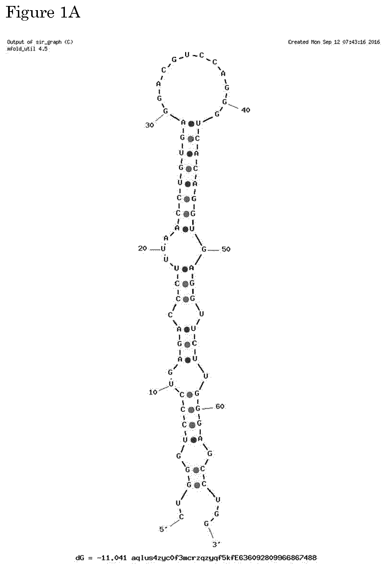

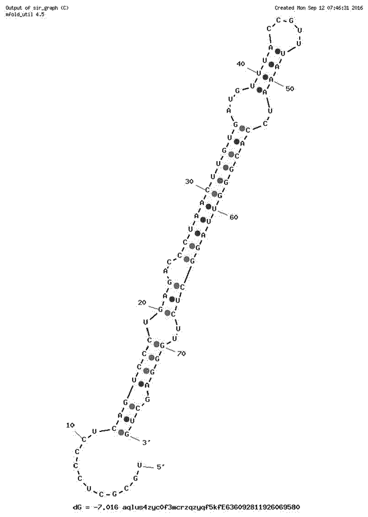

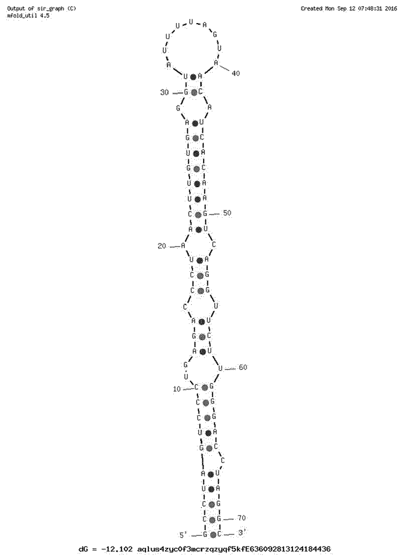

A miR-125 nucleic acid molecule for use according to the present invention may range in length from 18 to 100 nt (nucleotides) and may be mature miR-125 with a length of preferably from 19 to 25 nt and more preferably from 21 to 23 nt. Alternatively, the miR-125 nucleic acid molecules of the present invention may be provided as precursor miR-125 molecules with a length of from 50 to 100 nt and preferably from 65 to 95 nt. The nucleotide sequences of both the mature and the precursor microRNA-125 molecules are publicly available by reference to the database of the National Institute of Health (NIH), GenBank miR-125a(406910), miR-125b1(406911), miR-125b2(406912) and to miRBASE (http://microrna.sanger.ac.uk/), for example, miR-125a (Accession No. MI0000469 (ID: hsa-mir-125a) for the precursor form, and Accession Nos. MIMAT0000443 (ID: hsa-miR-125a-5p, Sequence 1) and MIMAT0004602 (ID: hsa-miR-125a-3p, Sequence 2) for the mature forms), miR-125b1 (Accession No. MI0000446 (ID: hsa-mir-125b-1) for the precursor form, and Accession Nos. MIMAT0000423 (ID: hsa-miR-125b1-5p, Sequence 3) and MIMAT0004592 (ID: hsa-miR-125b1-3p, Sequence 4) for the mature forms), miR-125b2 (Accession No. MI0000470 (ID: hsa-mir-125b-2) for the precursor form, and Accession Nos. MIMAT0000423 (ID: hsa-miR-125b2-5p, Sequence 5) and MIMAT0004603 (ID: hsa-miR-125b2-3p, Sequence 6) for the mature forms) (See Table 1 and Figure 1).

**Table 1: nucleotide sequences of mature miR-125 molecules**

| **Human sequences** | | **in mmu-miRbase** |
|---|---|---|
| Sequence 1 | hsa-miR-125a-5p | UCCCUGAGACCCUUUAACCUGUGA |
| Sequence 2 | hsa-miR-125a-3p | ACAGGUGAGGUUCUUGGGAGCC |
| Sequence 3 | hsa-miR-125b-5p | UCCCUGAGACCCUAACUUGUGA |
| Sequence 4 | hsa-miR-125b-1-3p | ACGGGUUAGGCUCUUGGGAGCU |
| Sequence 5 | hsa-miR-125b-2-5p | UCCCUGAGACCCUAACUUGUGA |

miR-125a>hsa-mir-125a MI0000469 
UGCCAGUCUCUAGGUCCCUGAGACCCUUUAACCUGUGAGG
ACAUCCAGGGUCACAGGUGAGGUUCUUGGGAGCCUGGCGUCUGGCC
miR-125b-1 >hsa-mir-125b-1 MI0000446 
UGCGCUCCUCUCAGUCCCUGAGACCCUAACUUGUGAUGUUU
ACCGUUUAAAUCCACGGGUUAGGCUCUUGGGAGCUGCGAGUCGUGCU
miR-125b-2 >hsa-mir-125b-2 MI0000470

ACCAGACUUUUCCUAGUCCCUGAGACCCUAACUUGUGAGGU
AUUUUAGUAACAUCACAAGUCAGGCUCUUGGGACCUAGGCGGAGGGGA

It should be appreciated that the miR-125 nucleic acid molecule of the present invention include functional equivalents of the constituent nucleic acid molecules, that is, variants which show the same functions as those of intact microRNA-125 nucleic acid molecules although they are mutated by deletion, substitution or insertion of some nucleotide residues.

Further, the microRNA-125 nucleic acid molecules of the present invention may exist in single-stranded or double stranded forms. Mature microRNA molecules are primarily in single-stranded forms while precursor microRNAs are partially self-complementary to form double-stranded structures (for example, stem-loop structures). In an alternative embodiment, the nucleic acid molecules of the present invention may be in the form of RNA, DNA, PNA (peptide nucleic acid) or LNA (locked nucleic acid). The nucleic acid molecules of the present invention may be isolated or prepared using standard molecular biology techniques, e. g, chemical synthesis or recombinant technology, or may be commercially available.

In addition to the microRNA-125 (mir-125) nucleic acid molecules, the anticancer composition of the present invention may comprise a material capable of improving the expression of the microRNA-125 in cells, such as synthetic or natural compounds or proteins, etc.

In one embodiment, the composition comprises an RNA sequence essentially corresponding to the RNA sequence of miR-125a, miR-125b1 and/or miR-125b2, or the complement sequence thereof. In a specific aspect, the RNA sequence essentially corresponds to the RNA sequence of miR-125a, or the complement thereof.

The term "RNA sequences corresponding substantially to miRNAs" as used herein refers to RNA sequences which are sufficient homologue to the RNA sequences of the miR-125a, miR-125b1 or miR-125b2, or the complement sequence thereof, respectively, but allow for derivative molecules of the miRNAs, such as pre- and pri-miRNA or may comprise modified sequences or modified nucleotides or backbone modifications but nonetheless fulfil the criteria that i) if the nucleic acid is an antagonist of the miRNA, the nucleic acid binds (or hybridizes) sufficiently strongly and/or specifically to the miRNA, pre-miRNA or pri-miRNA to thereby inhibit or reduce its activity and ii) if the nucleic acid is an agonist of the miRNA, the nucleic acid mimics or has the biological activity or a significant portion thereof, of the wild-type miRNA.

It is within the meaning of the invention that the sequences of the miRNAs miR-125a, miR-125b1 or miR-125b2 or the complement sequences thereof may also comprise a sequence that is the corresponding sequence of the respective pre-miRNA or pri-miRNA. When referring to "comprise a sequence", it is understood that in certain embodiments, "consisting essentially of" or "consisting of" are also included. For example, in certain embodiments, a nucleic acid comprises, consists essentially of or consists of a nucleotide sequence that is at least about 50%, 60%, 70%, 80%, 85%, 90%, 95%, 96%, 97%, 98%, 99% or 100% to that of the miRNA, pre-miRNA or pri-miRNA or the complement thereof. In certain embodiments, a nucleic acid comprises a nucleotide sequence that differs (by substitution, addition or deletion) from that of a miRNA, pre-miRNA or pri-miRNA in about 1, 2, 3, 4, 5, 6, 7, 8, 9, 10 or more nucleotides.

Delivery of oligonucleotides and/or expression vectors to a target cell can be achieved in a variety of ways. In some embodiments, a transfection agent is used. A transfection agent, or transfection reagent or delivery vehicle, is a compound or compounds that bind(s) to or complex(es) with oligonucleotides and polynucleotides, and enhances their entry into cells. Examples of transfection reagents include, but are not limited to, cationic liposomes and lipids, polyamines, calcium phosphate precipitates, polycations, histone proteins, polyethylenimine, polylysine, and polyampholyte complexes. Transfection reagents are well known in the art. Transfection reagents suitable for delivery of miRNA are e.g. HiPerfect (Qiagen) and NeoFX.<™> transfection agent (Ambion), which can be used to transfect a variety of cell types with miRNA. miRNAs can be readily electroporated into primary cells without inducing significant cell death. In addition, miRNAs can be transfected at different concentrations. Transfection agents may also be used to associate functional groups with a polynucleotide. Functional groups can include cell targeting moieties, cell receptor ligands, nuclear localization signals, compounds that enhance release of contents from endosomes or other intracellular vesicles (such as membrane active compounds), and other compounds that alter the behavior or interactions of the compound or complex to which they are attached (interaction modifiers). In other embodiments of the invention, the oligonucleotides enter into cells without a transfection agent.

To increase stability and/or optimize delivery of miRNAs or complement thereof, modified nucleotides or backbone modifications known in the art can be utilized. For example, modified nucleotides may include: linked nuclear acid (LNA), 2 -O-Me nucleotides, 2'-O-methoxyethyl, and T fluoro. Backbone modifications include, for example, phosphorothioate and phosphate. A 3' terminal cholesterol group appears to aid delivery of oligonucleotides like miRNAs to cells.

In accordance with an embodiment of the present invention, the composition comprises an expression vector carrying the miR-125 (mir-125). The microRNA-125 nucleic acid molecules of the present invention can be introduced into cells using DEAE-dextran-, nucleoprotein- or liposome-mediated DNA transfection. In this regard, the microRNA-125 nucleic acid molecules may be anchored at a carrier allowing for the effective delivery of nucleic acid molecules into cells. Preferably, the carrier is a vector, whether viral or non-viral. Examples of viral vectors useful in the present invention include vectors derived from lentivirus, retrovirus, adenovirus, herpes virus and avipox virus, preferably from lentivirus. Lentivirus, a kind of retrovirus, can productively infect both dividing and non-dividing cells because its pre-integration complex (virus "shell") can get through the nucleopores or intact membrane of the nucleus of the target cell.

In another embodiment of the invention, the composition comprises at least one agent capable of inhibiting a protein target of miR-125a, miR-125b1 or miR-125b2, said protein target comprising in its encoding RNA sequence the 6-mer seed region sequence 5'-CCUGAG -3'. Preferably, the target comprises in its RNA sequence the 7-mer 5'-CCCUGAG -3' or the 7-mer 5'-UCCCUGAG -3'. The composition may comprise at least two agents, each agent capable of inhibiting a distinct target of miR-125a, miR-125b1 or miR-125b2.

Suitable agents for use in the present invention include those capable of inhibiting one or more protein targets selected from the group consisting of PPP4R3A, PTPN1, PTPN7, MAPK14, VPS4B, SYVN1, M6PR, IL16, TXNRD1, GBP2, GBP5, GBP7, ERCC6, MAP2K7, MAP3K1, MUL1, BAK1, DFFA, DUSP3, MEF2C, RPS6KA1, STRADA, PPP2R1B, RELA, HLA-A, BAZ2A, DNMT3B and HIST2H3A, wherein each protein target is designated by its Gene Symbol numbers, as found in the database of the HUGO Gene Nomenclature Committee (HGNC). Table 2 below also lists the corresponding Gene Alias and NCBI Gene ID numbers for each of the target proteins.

Interestingly, it was found that many miR-125 target proteins are almost exclusively involved in cell signalling or intracellular trafficking and protein degradation. The best-characterized direct miR-125 target is MAPK14 (p38 MAP kinase). Other direct miR-125a targets included three protein tyrosine phosphatases (Ptpn1, Ptpn7, and Smek1, all known to suppress cytokine signalling and negatively regulate MAP kinases), a ubiquitin ligase (Syvn1, shown to block differentiation of embryonic stem cells (Yagishita et al., 2005), and Vps4b and M6pr involved in transport of protein to vesicles, or sorting vesicles and their lysosomal degradation). In addition, IL-16 and Txnrd1 are involved, the latter gene playing a role in protection against oxidative stress.

**Table 2: Gene Symbol numbers, the corresponding Gene Alias and NCBI Gene ID numbers for each of the miR-125 target proteins.**

| Gene_Symbol | Gene_Alias | Gene ID (NCBI) |
|---|---|---|
| PPP4R3A | Smek1 | 55671 |
| PTPN1 | Ptpn1 | 5770 |
| PTPN7 | Ptpn7 | 5778 |
| MAPK14 | P38MAPK, CSE | 1432 |
| VPS4B | Vps4b | 9525 |
| SYVN1 | Syvn1 | 84447 |
| M6PR | M6pr | 4074 |
| IL16 | IL16 | 3603 |
| TXNRD1 | Txnrd1 | 7296 |
| GBP2 | Gbp2 | 2634 |
| GBP5 | Gbp5 | 115362 |
| GBP7 | Gbp7 | 388646 |
| ERCC6 | Ercc6 | 2074 |
| MAP2K7 | Map2k7 | 5609 |
| MAP3K1 | Map3k1 | 4214 |
| MUL1 | Mul1 | 79594 |
| BAK1 | Bak1 | 578 |
| DFFA | Dffa | 1676 |
| DUSP3 | Dusp3 | 1845 |
| MEF2C | Mef2c | 4208 |
| RPS6KA1 | Rps6ka1 | 6195 |
| STRADA | Strada | 92335 |
| PPP2R1B | Ppp2r1b | 5519 |
| RELA | Rela | 5970 |
| HLA-A | Hla-A | 3105 |
| BAZ2A | Baz2a | 11176 |
| DNMT3B | Dnmt3b | 1789 |
| HIST2H3A | Hist2h3a | 333932 |

In one preferred embodiment, a composition in accordance with the invention comprises an inhibitor of MAPK14 (p38MAPK). Such inhibitors are known in the art. For example, the composition comprises the p38 inhibitor SB-203580 (4-[5-(4-Fluorophenyl)-2-[4-(methylsulfonyl)phenyl]-1H-imidazol-4-yl]pyridine); and/or SKF-86002 (Calbiochem).

In another preferred embodiment, the agent is an inhibitor of M6PR (cation dependent mannose-6-phosphate receptor), which is a member of the P-type lectin family. P-type lectins play a critical role in lysosome function through the specific transport of mannose-6-phosphate-containing acid hydrolases from the Golgi complex to lysosomes. M6PR functions as a homodimer and requires divalent cations for ligand binding. Suitable M6PR inhibitors for use in accordance with the invention include PXS25, or a pro-drug thereof, like PXS64.

The invention also provides a method for enhancing the mobilization of hematopoietic stem cells (HSCs) from the bone marrow to the peripheral blood in a subject, comprising administering to said subject an effective amount of a composition comprising a microRNA-125 (miR-125) nucleic acid molecule, or the complement sequence thereof, and/or an agent capable of inhibiting a (direct) target of miR-125, said target comprising in its RNA sequence the seed sequence 5'-CCUGAG-3'. For example, the method comprises administering an RNA sequence corresponding essentially to the RNA sequence of miR-125a, miR-125b1 or miR-125b2, or the complement sequence thereof, and/or an agent capable of inhibiting a target of miR-125a, miR-125b1 or miR-125b2, said target comprising in its RNA sequence the seed sequence 5'-CCUGAG-3'.

The term "administration", as used herein, is intended to mean the introduction of the pharmaceutical composition of the present invention to a subject using any appropriate method. In one embodiment, it comprises the delivery of the microRNA-125 nucleic acid molecule using viral or non-viral technology or by the transplantation of cells expressing microRNA-125. As long as it ensures the arrival of the composition of the present invention to a tissue of interest, any route may be taken for administration. For example, the composition of the present invention may be administered orally, rectally, topically, intravenously, intraperitoneally, intramuscularly, intraarterially, transdermally, intranasally, intrathoracically, intraocularly, or intradermally. Preferably, the anticancer composition of the present invention may be administered locally into cancer tissues.

The treatment method of the present invention includes administering the HSC-mobilizing composition of the present invention in a pharmaceutically effective amount. It will be apparent to those skilled in the art that the suitable total daily dose may be determined by an attending physician within the scope of sound medical judgment. The specific therapeutically effective dose level for any particular patient may vary depending on a variety of factors, including the kind and degree of desired reaction, the specific composition, including the use of any other agents according to the intended use, the patient's age, weight, general health, gender, and diet, the time of administration, route of administration, and rate of the excretion of the composition; the duration of the treatment; other drugs used in combination or coincidentally with the specific composition; and like factors well known in the medical arts. Accordingly, the effective amount of the composition suitable for the purpose of the present invention is preferably determined in full consideration of the above-mentioned factors. As described herein below, in some case, the HSC-mobilizing composition of the present invention may also be administered in combination with well-known HSC mobilizing so as to afford increased mobilization of HSCs into the peripheral blood.

Preferably, the subject to be treated in accordance with the invention is a human subject. For example, the subject exhibits a hematopoietic deficit from chemotherapy or radiation therapy. In one embodiment, the subject has a condition selected from the group consisting of aplastic anemia, leukemia and drug-induced anemia. In another embodiment, the subject is a transplantation recipient. Of course, the invention is also advantageously used to enhance HSC mobilization in a healthy stem cell donor.

A composition of the invention is particularly effective in sensitizing HSCs to "routine" or "conventional" stem cell mobilization regimes. Therefore, in a preferred aspect, the subject is undergoing a further stem cell mobilization regime concurrently with or after administering the miR-125 nucleic acid and/or the at least one agent capable of inhibiting a target of miR-125. For example, said further stem cell mobilization regime comprises administering a growth factor or a chemokine receptor antagonist. The growth factor may be selected from the group consisting of G-CSF, GM-CSF, IL-3, GM-CSF/IL-3 fusion proteins, FLK-2/FLT-3 ligand, stem cell factor, IL-6, IL-11, TPO and combinations thereof. Preferably, the growth factor is G-CSF. Preferred chemokine receptor antagonists include CXCR4 antagonists, such as AMD3100 (also known as Plerixafor).

The invention also provides a "HSC-mobilizing composition", the composition comprising (i) a microRNA-125 (miR-125) nucleic acid molecule, or the complement sequence thereof, and/or an agent capable of inhibiting a (direct) target of miR-125, said target comprising in its RNA sequence the seed sequence 5'-CCUGAG-3', and (ii) a further stem cell mobilization agent. For example, said further stem cell mobilization agent comprises a growth factor or a chemokine receptor antagonist. Exemplary growth factors in a composition of the invention include G-CSF, GM-CSF, IL-3, GM-CSF/IL-3 fusion proteins, FLK-2/FLT-3 ligand, stem cell factor, IL-6, IL-11, TPO and combinations thereof. Preferably, the invention provides a composition comprising (i) a miR-125 nucleic acid molecule, or the complement sequence thereof, and/or an agent capable of inhibiting a (direct) target of miR-125, said target comprising in its RNA sequence the seed sequence 5'-CCUGAG-3', and (ii) G-CSF. In another preferred aspect, the composition comprises (i) a miR-125 nucleic acid molecule, or the complement sequence thereof, and/or an agent capable of inhibiting a (direct) target of miR-125, said target comprising in its RNA sequence the seed sequence 5'-CCUGAG-3', and (ii) a chemokine receptor antagonist, preferably a CXCR4 antagonist, more preferably AMD3100 (Plerixafor).

In an embodiment, the HSC-mobilizing composition comprising one or more miR-125 nucleic acid molecules in accordance with the present invention may further comprise and be formulated with a pharmaceutically acceptable vehicle. The term "pharmaceutically acceptable vehicle", as used herein, is intended to refer to a carrier or a diluent which does not destroy the pharmaceutical activities and properties of the ingredient without the irritation of the subject to be treated. For use in liquid formulations of the composition of the present invention, the pharmaceutically acceptable vehicle is preferably suitable for sterilization and living body. The active ingredient of the present invention may be formulated with one selected from among saline, sterile water, Ringer's solution, buffered saline, albumin injection, dextrose solution, maltodextrose solution, glycerol, ethanol and combinations thereof, and if necessary, in combination with another conventional additives including antioxidants, buffer, bacteriostatic agents, etc. Alternatively, the composition of the present invention may be formulated into injections, pills, capsules, granules, or tablets with diluents, dispersants, surfactants, binders and/or lubricants.

The HSC-mobilizing composition in accordance with the present invention may be formulated into any dosage form, whether oral or non-oral. The pharmaceutical formulations according to the present invention may be administered via oral, rectal, nasal, topical (including bolus and sublingual), transdermal, vaginal, or parenteral (including intramuscular, subcutaneous and intravenous) routes or by inhalation or insufflation.

Examples of the oral dosage forms formulated with the composition of the present invention include tablets, troches, lozenges, water-soluble or oil suspensions, powders, granules, emulsions, hard or soft capsules, syrups or elixirs. For tablet or capsule formulations, useful are additives including a binder, such as lactose, saccharose, sorbitol, mannitol, starch, amylopectin, cellulose or gelatin, an excipient such as dicalcium phosphate, a disintegrant such as corn starch or sweet potato starch, and an lubricant, such as magnesium stearate, calcium stearate, sodium stearyl fumarate, sodium or polyethylene glycol. In addition to these additives, a liquid carrier such as fat oil may be used for capsule formulations.

For use in parenteral administration, the composition of the present invention may be formulated into injections via subcutaneous, intravenous or intramuscular routes, suppositories, or sprays via inhalation, such as aerosols. Injections may be prepared by mixing the composition of the present invention with a stabilizer or buffer in water to give solutions or suspensions which are packaged in unit dosages such as ampules or vials. For suppositories, the composition of the present invention may be formulated with a conventional base such as cocoa butter or glyceride, or an enema. The composition of the present invention in the form of a water-dispersed concentrate or a wet powder may be formulated with a propellant to prepare an aerosol spray.

### LEGEND TO THE FIGURES

**Figure 1** **: nucleotide sequences miR-125.** Nucleotide sequences of miR-125 refer to NCBI GeneID Nos. miR-125a (406910; panel A), miR-125b1 (406911; panel B) and miR-125b2 (406912; panel C), and miRBASE (http://microrna.sanger.ac.uk/) accession numbers mir125a (MI0000151), mir125b1 (MI0000725) and mir125b2 (MI0000152).
**Figure 2****: MiR-125a overexpression in LT-HSC and progenitors leads to equal distribution across the skeleton oppositely to control LT-HSC.**
   A.) Schematic representation of 5 bone groups that were analyzed.
   B.) Bone marrow cellularity (expressed as a total number of nucleated cells) isolated from various skeletal locations and C) the total number of LT-HSC (L-S+K+CD150+48-) FACS-sorted from certain parts of the skeleton.
   D.) Skeletal distribution of barcoded clones in control LT-HSC, miR-125a overexpressing LT-HSC in E.) or progenitors in F.).
   G.) Summary of all clones identified in 1 mouse transplanted with control LT-HSC, 12 weeks post transplantation (dots in dark grey correspond to the clone represented by the second bar section starting from the bottom for each bar in panel D.),
   H.) same as G but for miR-125a overexpressing LT-HSC,
   I.) the same as G but for miR-125a overexpressing progenitors. Similar pattern was observed in another 1-2 mice/condition that were sacrificed 12 weeks post transplantation (data not shown).
   J.) Summary of the RSDs for clones found in mice transplanted with CV LT-HSC (n=2), miR-125a LT-HSC (n=3), miR-125a progenitors (n=2) at 12 weeks post transplantation. Horizontal lines indicate the mean value. The difference between the groups was assessed by two-tailed Mann-Whitney test (***, P<.0001).
   K.) The response of L-K+GFP+ progenitors to increasing concentration of G-CSF, measured as the number formed CFU-GM colonies, circles represent control, squares miR-125a overexpressing cells.
   L.) The clonogenic potential of peripheral blood cells from mice injected with suboptimal dose of G-CSF.

### EXPERIMENTAL SECTION

### Materials and methods

### Mice

C57/BL/6 (B6) mice were purchased from Harlan, C57BL/6SJL were bred in the Central Animal Facility of the University Medical Center Groningen.

### Purification and transduction of BM cells.

LSK48-150+ , LSK depleted from CD48-150+ cell sorting and transduction were performed as previously described ([1]). Briefly BM cells from bones of hind legs, spines and sterna of naive, female B6 mice were crushed and stained with antibodies against Sca-1, c-Kit, CD48, CD150, CD3ε, Gr1, Ter119 and B220. Cells were sorted using MoFlo XDP and MoFlo Astrios (Beckman Coulter) cell sorters and prestimulated in StemSpan medium (STEMCELL Technologies) supplemented with 300 ng/ml stem cell factor (SCF), 1 ng/ml Flt3 ligand (Amgen), and 20 ng/ml IL-11 (R&R Systems) for 24 h. Cells were transduced with viral supernatant containing barcoded MIEV or 633-miR125a vectors in RetroNectin-covered plates (Takara Bio Inc.) in the presence of 2 µg/ml polybrene (Sigma-Aldrich).

### Barcoded libraries

MIEV library was constructed as previously described ([2]). To obtain 633-miR125a library the vector containing miR-125 was cut with PacI and SalI, synthesized oligos (IDT) were hybridized, ligated into the vector and used for bacteria (DH5α) transformation. Bacterial colonies were grown in 2 ml culture, 4 colonies per tube, then preps were made and stored. All preps were tested by PCR for the barcode region, as well as restriction analysis was done to discriminate barcoded and unbarcoded vectors. Note the current barcode version had a structure: (PacI)-GAATGGNNACNNAGNN**CCATGG**NNCANNCGNNTCTGGCG-(SalI).

This included the NcoI restriction site within the barcode sequence (bold). In total we obtained 434 clones, that were mixed in equal concentrations and used for transductions.

### Bone marrow cell transplantation.

20-24 h after the first transduction cells were transplanted into lethally irradiated recipients (9 Gy) in the presence of 1 million radioprotective cells from B6 mouse. The efficiency of gene transfer, measured by flow cytometry (LSR-II, BD) was typically between 20 and 50%. Donors were 4 months old and recipient animals were 2-4 months old. Donor and GFP chimerism were determined in blood every 4 weeks. For secondary transplantations 5 million whole BM cells were transplanted into lethally irradiated recipients.

### Q-PCR

Mir-125a overexpression level was measured as previously described ([3]).

### G-CSF administration.

To test the mobilization rate in control and miR-125a overexpressing cells, we performed one injection of PEGylated G-CSF intraperitoneally (25 µg/mouse dissolved in PBS) 3 days prior the sacrifice. To confirm the efficiency of the mobilization we also injected 1 naive B6 mouse and scored CFUs in the blood sample.

### Colony unit forming cell assay (CFU-GM).

To test the clonogenic potential of control and miR-125a overexpressing cells non-treated with G-CSF we put 100 000 cells isolated from the spleen or bone marrow or 1 capillary (50 µL) of peripheral blood and scored colonies at day 7.

To analyze the sensitivity of control or miR-125a overexpressing cells we put 500 L-K+GFP+ cells/plate, exposed cells to various concentrations of G-CSF (0-10 ng/ml) and scored colonies at day 7.

In order to assess the sensitivity of cells with and without miR-125 OE we injected mice with one dose of G-CSF. On day 3 we isolated L-K+GFP+ cells and put them in CFU-GM assay, on day 7 we scored colonies.

### Isolation of L-K+ cells from different skeletal locations after transplantation.

Transplanted recipients were sacrificed by cervical dislocation under isoflurane anesthesia. Bones were isolated separately into five groups: 1) bones of fore limbs and sternum (front); 2) left femur, tibia and left part of the pelvic bone (left hind bone); 3) right femur, tibia and right half of pelvic bone (right hind bones); 4) bones of spine, 5) bones of skull. After removal of muscles, cells were isolated by crushing in lysis solution (NH₄Cl) and filtered through 100 -µm filter to remove debris. Nucleated cells were washed, counted and stained with an antibody cocktail. Cells were washed and resuspended in a 1 µg/ml solution of propidium iodide (PI). Antibodies were directed against c-Kit, a panel of lineage markers (Ter119 Gr1, B220, Mac1, CD3ε). Cells were sorted directly into tubes, spun down and used for further barcode analysis.

### Clonal analysis of L-K+ cells.

Genomic DNA was extracted from L-K+ cells as described previously ([4]) and barcoded DNA was amplified with primers against internal vector sequence ([2]). For deep sequencing barcode region from each cell sample was amplified with specially tagged eGFP primers and un-tagged reverse primer (Rev YY1iPCR 5'CCAAACCTACAGGTGGGGTCTTTCATTC3'). DNA products were pooled together and sequenced as a single sample in Illumina 2500 HiSeq machine, rapid run, 100 bp long, according to the protocol of the manufacturer.

### Clonal analysis of blood samples.

Clonal analysis in blood was performed as previously described ([5]). In brief blood samples were collected every 4 weeks for 4-6 months and at the moment of sacrifice. Erythrocytes were lysed in NH₄Cl buffer, and cells were stained with fluorophore-conjugated antibodies against Gr1, B220, CD3ε, Ter119, washed and resuspended in PI solution. Mature blood cells were purified by FACS. Viable (PI-) granulocytes (Gr1+ side scatter high), T cells (CD3ε), B cells (B220+) and erythroid cells (Ter119+) were sorted. Barcode sequence was determined as described above.

### Barcode data processing

Data extraction was performed using in-house developed scripts in Python, Perl, R and VBA, as recently described ([5]). Briefly high quality barcode reads were extracted using Motif Occurrence Detection Suite (MOODS; [6]). Barcodes were sorted by read frequencies from highest to lowest, and all minor barcodes frequencies different by a single nucleotide (one base distance) were merged with the dominant major barcode. Unique barcodes with frequencies under 10 were removed (technical threshold). All samples with <1,000 reads were excluded from further analysis.

### Statistical analysis.

For testing the statistical significance of variation of clone size among different bone groups, we calculated the relative standard deviation (RSD). For comparing RSD values between groups we used non-parametric Mann-Whitney test, that allows comparison of not-normally distributed data. P-values were calculated using Prism.

Correlation and linear regression analyses and p-values were derived using Prism (GraphPad Software), Python and R.

Barcodes were normalized, sorted from highest to lowest and used for the linear regression with Pearson correlation to count linked barcodes and therefore estimate the number of cells with single, double and higher vector copy number

To visualize changes in the contribution and rank order of barcodes in serial transplantation experiments we included barcodes present>10 in the primary donor contributing >0.5% to Gr1+ cells and analyzed their contribution in secondary recipients. For data visualization we used custom Python script and R river plot algorithm. We have mainly used correlation coefficients (Pearson) as a measure of similarity between samples. This function is quantitative and have particular limitations, nevertheless is well known and broadly used, and when properly used provides sufficient statistical information.

### RESULTS

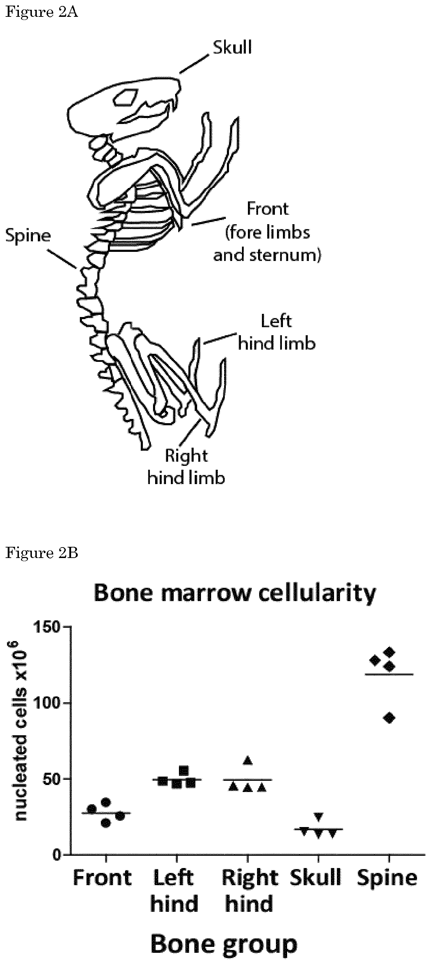

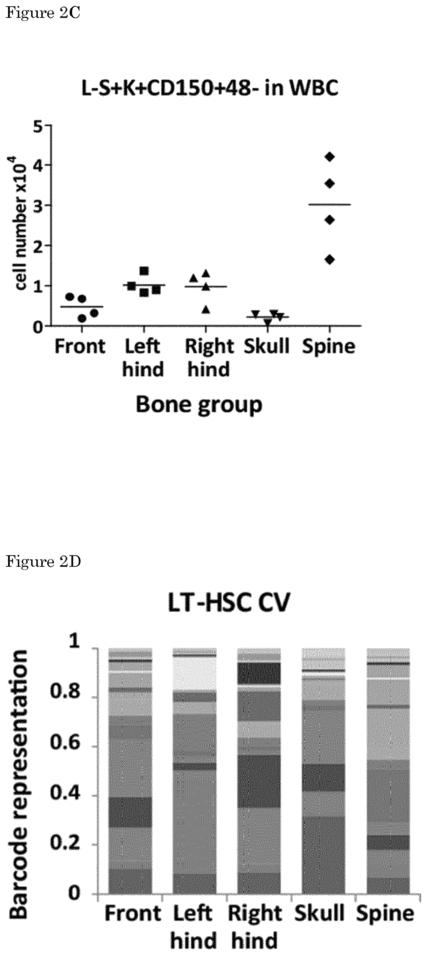

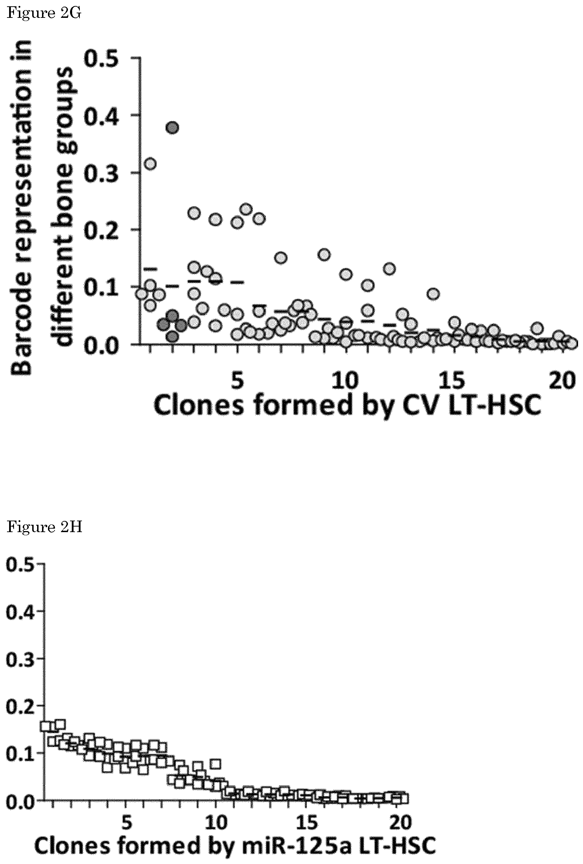

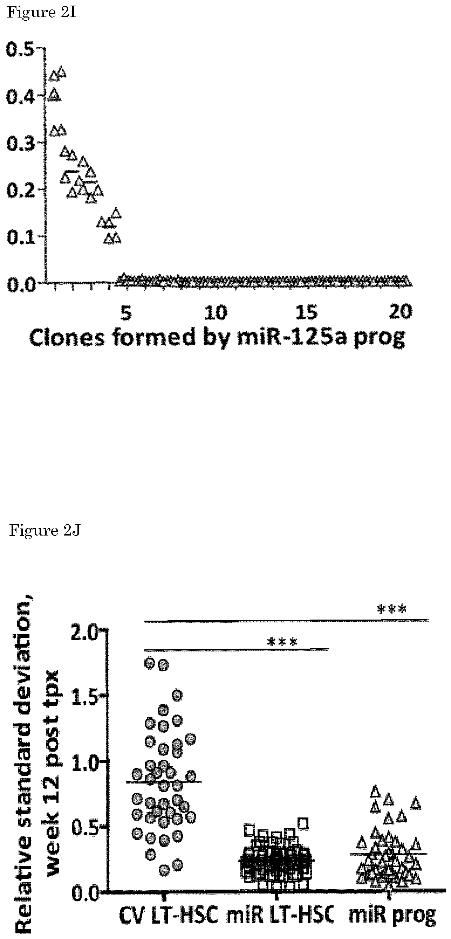

A DNA barcoded retroviral vector was used to mark individual hematopoietic stem cells prior to transplantation. We searched for clonal distributions at multiple sites throughout the skeleton (Figure 2A) after transplant. When HSCs were barcoded with an empty barcoded vector, hematopoietic stem cell clones were very asymmetrically distributed, indicating that normal stem cells do not migrate to a major extent (Figure 2D).

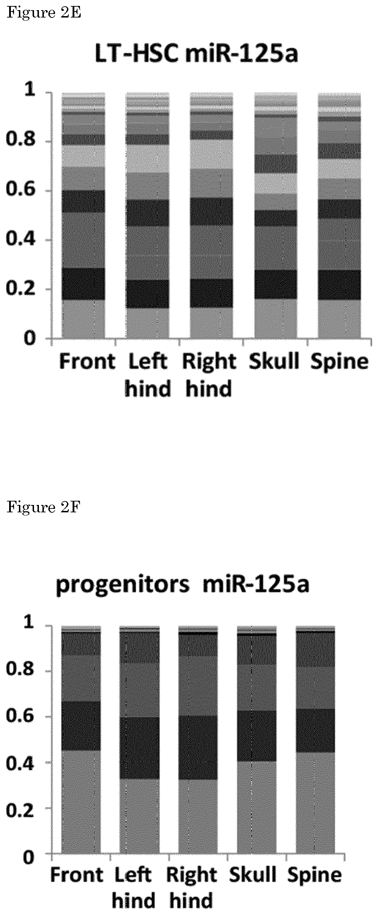

However, when miR125a was overexpressed in stem cells or progenitors using similarly barcoded vectors, a very different pattern of clonal distribution was observed (Figure 2E and 2F). Upon miR125a overexpression clonal distributions across the skeleton were virtually identical at any site tested. This indicates that miR125a-overexpressing stem cells migrate throughout the body with very substantial kinetics, and thus appear to cell-intrinsically, spontaneously, mobilize.

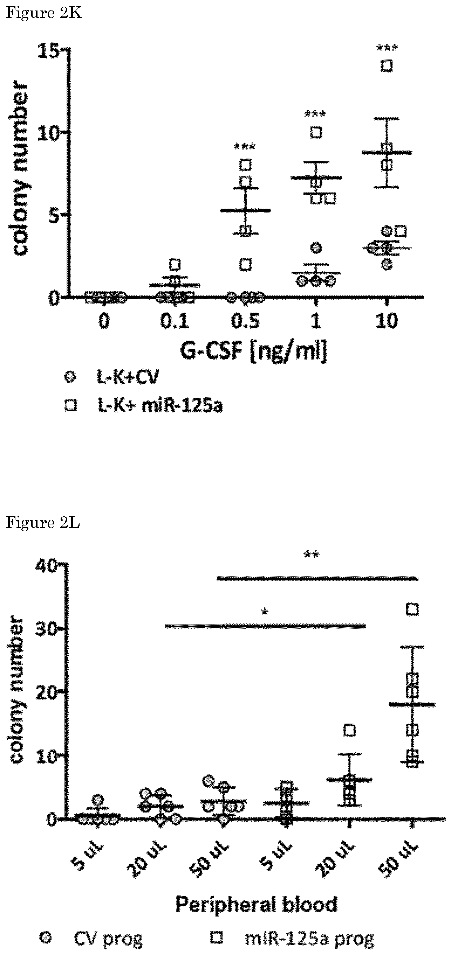

As stem cells can be mobilized from the bone marrow to the peripheral blood by administration of G-CSF, we speculated whether miR125a overexpression could render stem cells more responsive to G-CSF administration. Surprisingly, this was confirmed when we attempted to culture hematopoietic progenitors form the peripheral blood of G-CSF treated mice transplanted with stem cells overexpressing miR125a. Whereas, as expected, some mobilized progenitors could be cultured from blood obtained from mice transplanted with control stem cells, ∼10 fold more progenitors were present in the peripheral blood of mice transplanted with miR125a overexpressing stem cells (Figure 2L). These data demonstrate that miR125a overexpression leads to highly increased G-CSF induced stem cell mobilization.

We next asked whether miR125a overexpression would lead to G-CSF sensitization of stem- and progenitor cells. To test this, we overexpressed miR125a in hematopoietic stem cells, which were then transplanted to mice. Twenty weeks post-transplant, progenitor cells were isolated from these transplanted mice and cultured these in increasing doses of G-CSF (Figure 2K). Indeed, compared to control progenitor cells, which were transduced with an empty vector, miR125a overexpression led to a significant left-shifted G-CSF dose response curve, indicating an ∼20-fold increased G-CSF sensitivity upon miR-125a overexpression.

Collectively, these results demonstrate that repression of miR-125 target protein(s) increase G-CSF sensitivity by a factor 20, resulting in a strongly increased mobilization potential upon G-CSF administration.

### REFERENCES

1. Wojtowicz EE, Lechman ER, Hermans KG, Schoof EM, Wienholds E, Isserlin R, et al. Ectopic miR-125a Expression Induces Long-Term Repopulating Stem Cell Capacity in Mouse and Human Hematopoietic Progenitors. Cell Stem Cell. 2016;19: 383-396.
2. Gerrits A, Dykstra B, Kalmykowa OJ, Klauke K, Verovskaya E, Broekhuis MJ, et al. Cellular barcoding tool for clonal analysis in the hematopoietic system. Blood. 2010;115: 2610-2618.
3. Wojtowicz EE, Walasek MA, Broekhuis MJ, Weersing E, Ritsema M, Ausema A, et al. MicroRNA-125 family members exert a similar role in the regulation of murine hematopoiesis. Exp Hematol. 2014;42: 909-18.e1.
4. Verovskaya E, Broekhuis MJ, Zwart E, Weersing E, Ritsema M, Bosman LJ, et al. Asymmetry in skeletal distribution of mouse hematopoietic stem cell clones and their equilibration by mobilizing cytokines. J Exp Med. 2014;211: 487-497.
5. Verovskaya E, Broekhuis MJ, Zwart E, Ritsema M, van Os R, de Haan G, et al. Heterogeneity of young and aged murine hematopoietic stem cells revealed by quantitative clonal analysis using cellular barcoding. Blood. 2013;122: 523-532.
6. Korhonen J, Martinmaki P, Pizzi C, Rastas P, Ukkonen E. MOODS: fast search for position weight matrix matches in DNA sequences. Bioinformatics. 2009;25: 3181-3182.

## Claims

1. A composition comprising a microRNA-125 (miR-125) nucleic acid molecule, or the complement sequence thereof, and/or an agent capable of inhibiting a target of miR-125, said target comprising in its RNA sequence the seed sequence 5'-CCUGAG-3', for use in a method for enhancing the mobilization of hematopoietic stem cells (HSCs) from the bone marrow to the peripheral blood in a subject, preferably a human subject.

2. Composition for use according to claim 1, wherein said miR-125 nucleic acid molecule is miR-125a, miR-125b1 or miR-125b2, or the complement sequence thereof.

3. Composition for use according to claim 1, comprising at least one agent capable of inhibiting a target of miR-125, preferably wherein said miR-125 target comprises in its RNA sequence the seed sequence 5'-CCCUGAG -3'.

4. Composition for use according to any one of claims 1-3, comprising at least two agents, each agent capable of inhibiting a distinct target of miR-125a, miR-125b1 or miR-125b2.

5. Composition for use according to any one of the preceding claims, wherein said at least one agent is an inhibitor of a miR-125 target selected from the group consisting of PPP4R3A, PTPN1, PTPN7, MAPK14, VPS4B, SYVN1, M6PR, IL16, TXNRD1, GBP2, GBP5, GBP7, ERCC6, MAP2K7, MAP3K1, MUL1, BAK1, DFFA, DUSP3, MEF2C, RPS6KA1, STRADA, PPP2R1B, RELA, HLA-A, BAZ2A, DNMT3B and HIST2H3A.

6. Composition for use according to claim 5, wherein said agent is an inhibitor of MAPK14 (p38MAPK), preferably wherein said agent is SB253080.

7. Composition for use according to claim 5, wherein said agent is an inhibitor of M6PR, preferably wherein said agent is PXS25, or a pro-drug thereof, like PXS64.

8. Composition for use according to any one of the preceding claims, wherein the subject
(i) exhibits a hematopoietic deficit from chemotherapy or radiation therapy;
(ii) has a condition selected from the group consisting of aplastic anemia, leukemia and drug-induced anemia; and/or
(iii) wherein the subject is a transplantation recipient.

9. Composition for use according to any one of the preceding claims, wherein said subject is undergoing a further stem cell mobilization regime concurrently with or after administering the RNA sequence and/or agent capable of inhibiting a target of miR-125.

10. Composition for use according to claim 9, wherein said further stem cell mobilization regime comprises administering a growth factor or a chemokine receptor antagonist, preferably wherein the growth factor is G-CSF.

11. Composition for use according to any one of claims 1-7, wherein the subject is a healthy stem cell donor.

12. A composition comprising (i) a microRNA-125 (miR-125) nucleic acid molecule, or the complement sequence thereof, and/or an agent capable of inhibiting a (direct) target of miR-125, said target comprising in its RNA sequence the seed sequence 5'-CCUGAG-3', and (ii) a further stem cell mobilization agent.

13. Composition according to claim 12, wherein said further stem cell mobilization agent is a growth factor or a chemokine receptor antagonist.

14. Composition according to claim 13, wherein the growth factor is selected from the group consisting of G-CSF, GM-CSF, IL-3, GM-CSF/IL-3 fusion proteins, FLK-2/FLT-3 ligand, stem cell factor, IL-6, IL-11, TPO and combinations thereof, preferably wherein the growth factor is G-CSF.

15. Composition according to claim 14, wherein the chemokine receptor antagonist is a CXCR4 antagonist, preferably AMD3100 (Plerixafor).

16. A method for enhancing the mobilization of hematopoietic stem cells (HSCs) from the bone marrow to the peripheral blood in a subject, comprising administering to said subject an effective amount of a composition comprising (i) a microRNA-125 (miR-125) nucleic acid molecule, or the complement sequence thereof, and/or an agent capable of inhibiting a target of miR-125, said target comprising in its RNA sequence the seed sequence 5'-CCUGAG-3', and (ii) a further stem cell mobilization agent.
